# EUROPÄISCHE PATENTSCHRIFT

(11) **EP 3 125 743 B1**
(45) Veröffentlichungstag und Bekanntmachung des Hinweises auf die Patenterteilung: **23.03.2022**
(21) Anmeldenummer: 14715841.4
(22) Anmeldetag: 04.04.2014
(51) Int. Cl.: A61B 5/00, A61B 5/022

(54) **VERFAHREN ZUR BESTIMMUNG DES BLUTDRUCKS IN EINEM BLUTGEFÄSS SOWIE VORRICHTUNG ZUR DURCHFÜHRUNG DESSELBEN**
METHOD FOR DETERMINING BLOOD PRESSURE IN A BLOOD VESSEL AND DEVICE FOR CARRYING OUT SAID METHOD
PROCÉDÉ SERVANT À DÉTERMINER LA TENSION ARTÉRIELLE DANS UN VAISSEAU SANGUIN ET DISPOSITIF SERVANT À EFFECTUER CETTE DÉTERMINATION

(43) Veröffentlichungstag der Anmeldung: 08.02.2017
(73) Patentinhaber: Philips Medizin Systeme Böblingen GmbH, 71034 Böblingen (DE)
(72) Erfinder: PFEIFFER, Ulrich, 81667 München (DE); KNOLL, Reinhold, 94127 Neuburg (DE)
(74) Vertreter: Philips Intellectual Property & Standards
(86) Internationale Anmeldenummer: PCT/EP2014/000905
(87) Internationale Veröffentlichungsnummer: WO 2015/149822

(56) Entgegenhaltungen:
- EP-A1- 0 467 853
- EP-A1- 1 388 321
- WO-A2-2009/141171
- DE-A1- 3 533 912
- US-A1- 2013 211 208
- US-B1- 6 475 153

## Beschreibung

Bei der arteriellen Blutdruckmessung wird der Druck in einem arteriellen Blutgefäß eines Menschen oder Tiers gemessen. Er spielt in der medizinischen Diagnostik eine zentrale Rolle für eine Vielzahl von Erkrankungen, insbesondere des Herz-/Kreislaufsystems.

Weiterhin unterscheidet man die direkte, invasive Blutdruckmessung mittels eines mit dem Blut in einem Blutgefäß in direkter hydraulischer Verbindung stehenden Druckfühlers von der indirekten, nicht-invasiven Blutdruckmessung, die üblicherweise mit Hilfe einer pneumatischen Manschette an einer Extremität durchgeführt wird.

Bei der direkten Messung wird ein Blutgefäß, meist eine periphere Arterie, punktiert und ein Katheter eingebracht. Dieser wird über einen flüssigkeitsgefüllten Schlauch mit einem Drucksensor verbunden, über welchen sich die arterielle Blutdruckkurve auf einem Monitor darstellen lässt. Die Messung ist genau und bietet den Vorteil einer kontinuierlichen Überwachung, solange eine durchgehende und keine Blutgerinnsel oder Luftblasen enthaltene Flüssigkeitssäule zwischen Blutgefäss und Drucksensor gewährleistet ist und der Druckschlauch die erforderliche Steifigkeit aufweist, was in der klinischen Praxis nicht immer gegeben ist. Zusätzlich können die Herzfrequenz, der systolische, diastolische und der mittlere arterielle Blutdruck (SAP, DAP, MAP), die Pulsdruckvariation (PPV) sowie mittels Pulskonturverfahren das Herzzeitvolumen (PCCO) und die Schlagvolumenvariation (SVV) bestimmt werden. Da die Methode zeitaufwendig und invasiv ist, verursacht sie erheblich höhere Kosten und geht mit dem Risiko von Blutungen, Hämatomen, Thromboembolien, Infektionen und Nervenverletzungen einher. Sie wird vor allem zur Überwachung während einer Operation, auf Intensivstationen und im Herzkatheterlabor eingesetzt, außerhalb dieser Anwendungen jedoch in der Regel nicht.

Bei der indirekten arteriellen Druckmessung wird der arterielle Druck mit Hilfe eines Blutdruckmessgerätes an einer Extremität, meist am Arm, gemessen. Obwohl indirekte Messungen nicht so genau wie direkte Messungen des Blutdrucks sind, machen die leichte, schnelle, ungefährliche und kostengünstige Durchführung sie zum Mittel der Wahl bei den meisten medizinischen Anwendungen. Die manuelle oder automatisierte Messung kann auskultatorisch, palpatorisch und oszillatorisch durchgeführt werden.

Bei der auskultatorischen Messung wird eine pneumatische Druckmanschette am Oberarm bis oberhalb des erwarteten arteriellen Drucks aufgeblasen. Beim langsamen Absenken des Manschettendrucks kann das Auftreten und danach wieder das Verschwinden eines sogenannten Korotkov-Geräuschs mit Hilfe eines Stethoskops über der Arterie des Armes gehört werden. Der Wert, der bei erstmaliger akustischer Wahrnehmbarkeit des Korotkov-Geräuschs auf der Skala des Manometers abgelesen werden kann, entspricht dem oberen, systolischen Blutdruck. Der systolische Blutdruck ist in diesem Moment größer als der Druck in der Manschette. Der Druck in der Manschette wird mit geeigneter Geschwindigkeit weiter verringert. Unterschreitet dieser den diastolischen arteriellen Druck, verschwindet das Korotkov-Geräusch. Dieser Wert entspricht dem diastolischen Blutdruck. Die auskultatorische Messung dient nach wie vor als Referenzverfahren bei den nicht-invasiven Messverfahren.

Auch bei der palpatorischen Messung wird eine Druckmanschette am Oberarm angelegt. Beim erneuten Verringern des Manschettendrucks wird der Puls an der Arteria radialis getastet. Der Wert, der beim erstmals getasteten Puls auf der Skala des Messgerätes abgelesen werden kann, entspricht dem systolischen Blutdruck. Der diastolische Blutdruck kann auf diese Weise nicht ermittelt werden. Das Verfahren bietet sich insbesondere für eine Anwendung in einer lauten Umgebung oder dann, wenn ein Stethoskop nicht griffbereit wäre, an.

Bei der oszillatorischen Messung wird mit Hilfe einer Druckmanschette die Arterie am Oberarm oder Handgelenk oder Bein abgedrückt. Während die Luft langsam aus der Druckmanschette abgelassen wird, beginnt das Blut wieder durch die Arterie zu fließen. Hierbei können Schwingungen der Arterienwände, die durch den beginnenden Blutfluss ausgelöst werden, registriert werden. Diese Schwingungen, auch Oszillationen genannt, werden zuerst stärker, nehmen ab und klingen vollkommen ab, wenn das Blut wieder normal durch die Blutgefäße fließt. Die Schwingungen werden auf die Druckmanschette übertragen und führen so zu einem oszillierenden Zeigerausschlag des Manometers. Die Maxima und Minima des Zeigerausschlags entsprechen zeitlich dem systolischen und diastolischen Blutdruck. Aus dem Manschettendruck zum Zeitpunkt der maximalen Oszillationen und der Halbwertsbreite kann der systolische und diastolische Druck berechnet werden. Bei einer manuellen Messung lassen sich mit diesem Verfahren nur ungenaue Ergebnisse erzielen. Dieses Verfahren wird jedoch in automatischen Blutdruckmessvorrichtungen insbesondere auch bei dauerhafter Überwachung, z.B. intraoperativ und/oder postoperativ im Aufwachraum, eingesetzt. Dabei messen die Blutdruckmessvorrichtungen als Alternative zur kontinuierlichen invasiven Druckmessung den arteriellen Blutdruck des Patienten intermittierend in Intervallen von wenigen Minuten. Bei einer elektronischen Auswertung der ermittelten Schwingungen wird aus dem Kurvenverlauf der Schwingungen mathematisch mit Hilfe eines Algorithmus der systolische und diastolische Blutdruck berechnet.

Gemäß der EP 0 467 853 B1 ist in der Publikation "Possible determinants of pulse wave velocity in vivo", Masahiko Okada, IEEE Transactions on Biomedical Engineering, Vol. 35, Nr. 5, Mai 1988, Seiten 357 bis 361 offenbart, dass eine gewisse Korrelation zwischen der Pulswellengeschwindigkeit und dem systolischen und diastolischen Blutdruck feststellbar sei. Diese schwache geringe Korrelation soll jedoch nach dieser Lehre keine Bestimmung des Blutdrucks ermöglichen.

Darauf aufbauend wird in der EP 0 467 853 B1 ein Verfahren zur kontinuierlichen Ermittlung des Blutdrucks eines Patienten vorgeschlagen, bei dem mittels einer elektronischen Messvorrichtung, die zwei in einem definierten Abstand angeordnete Sensoren aufweist, zumindest eine sich zeitlich im Takt des Pulses ändernde Größe ermittelt wird, die ein Maß für die Strömungsgeschwindigkeit, die Durchflussmenge, das Volumen des arteriellen Blutes oder den Durchlassquerschnitt eines arteriellen Blutgefäßes ist, und eine zweite Größe ermittelt wird, die ein Maß für die Pulswellengeschwindigkeit einer durch einen Herzschlag bedingten Druckwelle in dem Blut in diesem Blutgefäß ist. Aus beiden Größen wird unter Einbeziehung von Kalibrationswerten der Blutdruck ermittelt. Dabei könnte beispielsweise ausgenutzt werden, dass der Blutdruck proportional zu der Strömungsgeschwindigkeit des Bluts ist. Als Proportionalitätsfaktor dient dabei der Quotient: kᵥ/r2, wobei kᵥ eine Konstante und r der Radius des betrachteten Gefäßabschnitts (bei angenommenem kreisförmigem Querschnitt) ist. Wäre r konstant, ließe sich der Blutdruck aus mehreren Messungen der Strömungsgeschwindigkeit direkt ermitteln. Da die Gefäßwand jedoch elastisch und r demnach nicht konstant, sondern hochvariabel ist, erfolgt die Verknüpfung mit der Messung der Pulswellengeschwindigkeit. Dabei wird ausgenutzt, dass sich das Elastizitätsmodul der Gefäßwand aus der Pulswellengeschwindigkeit ermitteln lässt.

Die Funktion der Sensoren bei dem in der EP 0 467 853 B1 offenbarten Verfahren kann optisch basiert sein. Dazu können diese jeweils einen als Lichtstrahler dienenden optoelektronischen Wandler, z.B. eine Laser-Leuchtdiode, und einen als Lichtempfänger dienenden optoelektronischen Wandler, z.B. eine Photodiode, aufweisen. Beide Sensoren können in eine Manschette integriert sein und zur Blutdruckermittlung, beispielsweise an einem der Unterarme eines Patienten, befestigt werden. Eine Kalibration der sensorbasierten Ermittlung des Blutdrucks kann, einem konventionellen, nicht-invasiven Verfahren nach Riva-Rocci entsprechend, mit einer aufblasbaren Manschette erfolgen. Dabei soll die Manschette an einer anderen Extremität des Patienten angelegt werden, als dies für die Sensor-Manschette vorgesehen ist, um eine Verfälschung der sensorbasierten Messungen zu vermeiden. Eine Kalibration kann einmal zu Beginn der sensorbasierten Langzeitmessung oder mehrfach nach jeweils einem definierten Zeitraum, z.B. täglich, erfolgen.

Die US 2013/0079648 A1 offenbart ein Verfahren und eine Vorrichtung zur Messung der Pulswellengeschwindigkeit in einer Ader, wobei mittels zweier Drucksensoren, die in einem definierten, kurzen Abstand in beispielsweise eine Manschette integriert sind, eine druckbedingte Aufweitung der Ader unterhalb der Sensoren detektiert werden. Die Pulswellenlaufzeit kann mittels der Sensoren ermittelt werden, wenn beispielsweise die Zeitdifferenz der Ermittlung eines der gleichen Pulswelle zugeordneten Druckmaximums durch die beiden Sensoren herangezogen wird. Aus der Pulswellenlaufzeit kann dann in Verbindung mit der bekannten Distanz zwischen den Sensoren auf die Pulswellenlaufzeit geschlossen werden.

In der US 2013/0079648A1 ist weiterhin offenbart, dass anhand der (beiden) Drucksensoren auch auf den Blutdruck geschlossen werden kann. Dabei wird die Spannung, die von den beiden Drucksensoren gemessen wird, anhand einer Transformationskoeffizientenmatrix jeweils in einen Wert für den Blutdruck umgerechnet. Als ermittelter Wert für den Blutdruck wird dann der Mittelwert aus den beiden Werten der beiden Drucksensoren genommen. Da die Transformationskoeffizientenmatrix im Allgemeinen nicht bekannt und mit der Zeit veränderlich ist, ist diese Methode praktisch nicht umsetzbar.

WO 2009/141171 A2 betrifft einen piezoelektrischen Sensor für die verbesserte Messung mechanischer Größen wie Kraft, Druck oder Messgrößen.

EP 1 388 321 A1 betrifft ein Verfahren und ein System zur Messung nicht-invasiv einen Blutdruck eines Patienten.

US 6 475 153 B1 betrifft einen optischen Sensor zur Erzeugung von Blutdruck betreffende Daten, um zweidimensionale Bilder der Oberfläche des Körpers des Patienten zu erhalten.

DE 35 33 912 A1 betrifft ein Sphygmomanometer.

Ausgehend von diesem Stand der Technik lag der Erfindung die Aufgabe zugrunde, ein konstruktiv möglichst einfach umsetzbares, einfach anwendbares und/oder den Patienten nicht beeinträchtigendes Verfahren zur kontinuierlichen nichtinvasiven Messung des Blutdrucks anzugeben.

Diese Aufgabe wird mittels eines Verfahrens gemäß Patentanspruch 1 gelöst. Eine Vorrichtung zur Durchführung eines solchen Verfahrens ist Gegenstand des Patentanspruchs 11. Vorteilhafte Ausführungsformen des Verfahrens und vorteilhafte Ausgestaltungen der Vorrichtung sind Gegenstand der weiteren Patentansprüche und ergeben sich aus der nachfolgenden Beschreibung der Erfindung.

Das erfindungsgemäße Verfahren basiert auf der Erkenntnis, dass, obwohl die in der EP 0 467 853 B1 beschriebene Korrelation zwischen der Pulswellengeschwindigkeit und dem Blutdruck als zu schwach bezeichnet wird, als dass daraus der Blutdruck ermittelt werden könnte, eine Ermittlung des Blutdrucks hierdurch gleichwohl ermöglicht wird. Das erfindungsgemäße Verfahren benötigt im Gegensatz zum in der EP 0467 853 B1 beschriebenen Verfahren kein zur Strömungsgeschwindigkeit proportionales Signal. Es reicht ein beliebiges sich synchron zum Pulsschlag veränderndes Signal aus. Es wurde nämlich erkannt, dass die Pulswellenlaufzeitänderung als zumindest funktional abhängig von und unter bestimmten Voraussetzungen bei nicht zu großen Veränderungen des Blutdruckverlaufs als umgekehrt proportional zur Blutdruckänderung angesehen werden kann. Insbesondere kann die mittlere Pulswellenlaufzeitänderung unter bestimmten Voraussetzungen als umgekehrt proportional zu der mittleren Blutdruckänderung angesehen werden. Insbesondere kann auch die Laufzeitänderung zum Systolen- oder Diastolen-Zeitpunkt unter bestimmten Voraussetzungen als umgekehrt proportional zu den systolischen oder diastolischen Blutdruckänderungen angesehen werden. Der funktionale Zusammenhang bzw. die Proportionalitätskonstante muss für jeden Probanden bzw. Patienten und ev. bei größeren physiologischen Änderungen durch Kalibrierung neu ermittelt werden. Vorteilhaft können während eines Pulsschlags auch mehrere Laufzeitänderungen und damit mehrere Druckänderungen bestimmt werden. Der aktuelle Blutdruck wird dann aus dem ursprünglichen mittels Kalibrierung bestimmten Blutdruck und der Blutdruckänderung bestimmt. Dieser zumindest funktionale Zusammenhang ermöglicht in Verbindung mit einer Kalibrierung mittels zumindest zwei quantitativen Werten für den Blutdruck, insbesondere des diastolischen Blutdrucks (DAP) und/oder des systolischen Blutdrucks (SAP) und/oder des mittleren Blutdrucks (MAP), ergebenden Messung eine Ableitung eines quantitativen Blutdruckverlaufs aus dem Blutdruck, insbesondere des DAP und/oder SAP und/oder MAP.

Demnach ist bei einem erfindungsgemäßen Verfahren zur Bestimmung eines Blutdrucks in einem Blutgefäß, bei dem in einem Messvorgang mittels mindestens zweier, in einem definierten Abstand zueinander angeordneter Sensoren eine Pulswellenlaufzeit ermittelt wird, vorgesehen, dass durch eine Kalibrierung aus der Pulswellenlaufzeitänderung der Blutdruck ohne Verwendung eines weiteren, zur Strömungsgeschwindigkeit proportionalen Signals ermittelt wird, wobei die Pulswellenlaufzeitänderung umgekehrt proportional zur Blutdruckänderung ist. Die Kalibrierung kann im einfachsten Fall der umgekehrt proportionalen Abhängigkeit mit mindestens zwei bekannten Druckwerten erfolgen, zum Beispiel mit dem mittleren Druck und dem diastolischen Druck aus einer Klemmdruckmessung.

Unter einer Klemmdruckmessung wird ein Verfahren verstanden, bei dem mittels einer an einer Extremität des Menschen oder Tieres, dessen Blutdruck bestimmt werden soll, angelegten Druckmanschette (insbesondere durch deren Aufblasen) ein Druck auf den entsprechenden Abschnitt der Extremität ausgeübt wird, der zunächst so groß gewählt wird, dass ein Blutfluss durch das betrachtete Blutgefäß in diesem Abschnitt der Extremität unterbrochen wird. Der mittels der Druckmanschette aufgebrachte Druck wird anschließend kontrolliert reduziert und aus dabei auftretenden Vorgängen auf den Blutdruck(verlauf) geschlossen. Umgekehrt kann auch schon während der mit konstanter oder variierender Geschwindigkeit vorgenommenen Inflation der Druckmanschette auf den Blutdruck(verlauf) rückgeschlossen werden. Beispielsweise können die bereits beschriebenen auskultatorischen und palpatorischen Klemmdruckverfahren erfindungsgemäß zur Kalibrierung genutzt werden. Bevorzugt bietet sich jedoch das bereits beschriebene oszillatorische Klemmdruckverfahren an, da vorzugsweise eine vollständig automatische Auswertung der dabei ermittelten Messwerte und deren Nutzung zur Kalibrierung der sensorbasierten Messungen erfolgen soll.

Bei der Durchführung des erfindungsgemäßen Verfahrens kann insbesondere auch bei nicht mehr erfüllten Voraussetzungen der umgekehrt proportionalen Abhängigkeit, weiter vorgesehen sein, dass eine aus der Klemmdruckmessung abgeleitete Pulskurvenform (d.h. dem Verlauf des Blutdrucks über einen Herzschlag) genutzt wird, um eine Transferfunktion zu bestimmen. Die Transferfunktion wird durch Rückfaltung eines gemessenen Sensorsignals mit dem aus anderen Messverfahren bekannten Pulskurvensignal gewonnen. Mittels dieser Transferfunktion kann bei späteren Messungen ohne ein aus einem anderen Messverfahren bekannten Pulskurvensignal aus dem Verlauf der mittels der Sensoren ermittelten Signale durch Faltung eine Pulskurvenform ermittelt werden. Zusätzlich dazu kann im Rahmen der Kalibrierung die aus dem Signalverlauf ermittelte Pulskurvenform quantitativ definiert werden.

Ein bevorzugtes Verfahren zur Bestimmung einer Pulskurvenform ausgehend von einer Klemmdruckmessung ist in der internationalen Patentanmeldung PCT/EP2014/000031 offenbart. Dieses Verfahren beruht darauf, mehrere Pulskurven bei verschiedenen konstanten Klemmdrücken oder kontinuierlich ansteigendem oder fallendem Klemmdruck aufzuzeichnen, wobei jede Pulskurve mit einer vom Klemmdruck abhängigen Funktion gewichtet wird. Die Summe der gewichteten Pulskurven kann dann als gute Näherung für den unskalierten invasiven Blutdruckverlauf, d.h. die hier relevante Pulskurvenform, angenommen werden. Vorzugsweise kann die Gewichtung in Abhängigkeit des Abstands des invasiven Blutdrucks vom Klemmdruck erfolgen, wobei als Ausgangswert für die Durchführung des Verfahrens ein Schätzwert für den invasiven Blutdruck genutzt werden kann. Der gesamte Inhalt der PCT/EP2014/000031 wird durch Bezugnahme zum Inhalt der vorliegenden Beschreibung gemacht.

Diese Ermittlung einer Pulskurvenform mittels einer zuvor ermittelten Transferfunktion kann somit insbesondere auch dann erfolgen, wenn gerade keine Klemmdruckmessung durchgeführt wird. Demnach kann vorzugsweise vorgesehen sein, dass eine Mehrzahl von Messvorgängen durchgeführt wird, wobei Kalibrierungen nur nach (jeweils) mehreren Messvorgängen durchgeführt werden. Ein Vorteil des erfindungsgemäßen Verfahrens liegt dann darin, dass die vorzugsweise mittels Klemmdruckmessung erfolgende Kalibrierung nicht für jeden Messvorgang durchgeführt werden muss, sondern die sich dabei ergebenden Messwerte zur Kalibrierung für mehrere Messvorgänge genutzt werden können. Demnach kann eine kontinuierliche Ermittlung des Blutdrucks mittels der Sensoren erfolgen, während eine Pulskurvenform, die zur Kalibrierung der sensorbasierten Messwerte herangezogen und durch insbesondere eine Klemmdruckmessung ermittelt wird, nur intermittierend ermittelt wird. Dabei kann der Zeitraum zwischen jeweils zwei Kalibrierungsmessungen variabel gewählt werden. Zu beachten ist dabei, dass eine relativ häufige Kalibrierung die Genauigkeit der sensorbasierten Ermittlung des Blutdrucks zwischen den Klemmdruckmessungen verbessern kann, jedoch wegen des (bei einer Klemmdruckmessung) kurzzeitig aufzubringenden Klemmdrucks auf eine Extremität des wachen Patienten in der Regel als weniger angenehm oder auch unangenehm wahrgenommen wird. Die Kalibrierungsmessungen sollten daher so selten wie möglich aber so häufig, wie für das Erreichen einer ausreichenden Ermittlungsgenauigkeit nötig ist, durchgeführt werden. Beispielsweise kann vorgesehen sein, Kalibrierungsmessungen in Abständen von zwei bis fünfzehn Minuten, bspw. in Abständen von ca. fünf oder ca. zehn Minuten durchzuführen. Selbstverständlich besteht auch die Möglichkeit, die Zeiträume zwischen jeweils zwei nacheinander erfolgenden Kalibrierungsmessungen unterschiedlich lang oder Serien von aufeinanderfolgenden Kalibrierungsmessungen in variablen Abständen vorzusehen.

Es ist vorgesehen, dass der Druckverlauf bzw. die Pulswelle optisch ermittelt wird. Dabei werden mittels (jeweils) eines Leuchtmittels der Sensoren Lichtpulse in Richtung des Blutgefäßes ausgestrahlt, wobei mittels eines Detektors der Sensoren ein von dem Blut in dem Blutgefäß reflektierter Anteil und/oder ein durch das Blut in dem Blutgefäß transmittierter Anteil der Lichtpulse (bzw. eine Änderung desselben) detektiert wird. Schwankungen im Bereich der Herzfrequenz des detektierten Messsignalverlaufs der mindestens zwei Sensoren können dabei dem sich lokal verändernden Blutdruck zugeordnet werden. Die Verwendung von pulsierenden Leuchtmitteln und eine Detektion im aktivierten und nichtaktivierten Zustand des Leuchtmittels ermöglicht in bekannter Weise die Separation von Fehlersignalen z.B. Fremdlicht.

Eine Vorrichtung zur Durchführung eines erfindungsgemäßen Verfahrens umfasst zumindest die Sensoren, eine Druckmanschette zur Durchführung der Klemmdruckmessung und eine Auswerteeinheit, wobei die Sensoreinheit und gegebenenfalls auch die Auswerteeinheit in die Druckmanschette integriert ist/sind. Dadurch können sich ein kompakter Aufbau der erfindungsgemäßen Vorrichtung sowie eine einfache Handhabung ergeben. Problematisch dabei kann unter gewissen Umständen der relativ geringe Abstand zwischen den mindestens zwei Sensoren sein, der zu einer entsprechend kurzen (zu bestimmenden) Pulswellenlaufzeit führt.

Insbesondere um solche kurzen Pulswellenlaufzeiten ausreichend genau kontinuierlich messen zu können, ist vorgesehen, dass ein Zeitversatz zwischen der Aktivierung (d.h. dem Aussenden eines Lichtpulses und dem Detektieren eines reflektierten und/oder transmittierten Anteils dieses Lichts) der mindestens zwei optischen Sensoren (d.h. einem Aktivieren eines ersten (dem stromauf gelegenen) der Sensoren und dem darauffolgenden, zeitversetzten Aktivieren des zweiten Sensors) derart (insbesondere kontinuierlich) geregelt wird, dass für beide Sensoren möglichst gleiche relative Messwerte erzielt werden und demnach der Zeitversatz im Wesentlichen der Pulswellenlaufzeit entspricht. Dabei werden unter gleichen "relativen" Messwerten solche verstanden, die im Wesentlichen die gleiche Stelle der hinsichtlich der Kurvenform vergleichbaren Messwertverläufe darstellen. Dabei können die gleichen "relativen" Messwerte durchaus unterschiedliche absolute Messwerte darstellen. Dies kann insbesondere darin begründet sein, dass infolge der unterschiedlichen Positionierung der mindestens zwei Sensoren (und dem damit möglicherweise einhergehenden unterschiedlichen Abstand zu dem jeweiligen Abschnitt des betrachteten Blutgefäßes und/oder des unterschiedlichen Aufbaus des zwischen den beiden Sensoren und den entsprechenden Blutgefäßabschnitten liegenden Gewebes) auch bei gleich starken von den beiden Sensoren ausgesendeten Lichtpulsen unterschiedlich große reflektierte und/oder transmittierte Anteile detektiert werden. Diese hinsichtlich der absoluten Werte unterschiedlichen Messwertverläufe können deren vorausgehende Normierung ggf. sinnvoll machen.

Zur Vereinfachung der Regelung der zeitversetzten Aktivierung der beiden Sensoren kann vorgesehen sein, dass die (jeweilige) Zeitverzögerung anhand charakteristischer und somit gut identifizierbarer und daher gut vergleichbarer Werte der Messwertverläufe ermittelt wird. Als charakteristische Werte können insbesondere die Minima (die dem daraus zu ermittelnden diastolischen Blutdruck entsprechen können), die Maxima (die dem daraus zu ermittelnden systolischen Blutdruck entsprechen können) und/oder die Stellen des maximalen Messwertanstiegs herangezogen werden.

Der Zeitversatz kann grundsätzlich analog oder digital, beispielsweise mit einer Auflösung von zwischen 3 und 15 ns, insbesondere 5 ns, geregelt werden.

Zur Ermittlung einer eventuellen Regelungsabweichung des Zeitversatzes für einen anstehenden Messvorgang kann die zeitliche Verzögerung zwischen der Ermittlung desselben charakteristischen Werts der von den Sensoren in einem vorausgegangenen Messvorgang ermittelten Messwertverläufe genutzt werden. Vorzugsweise kann vorgesehen sein, dass die Verzögerung ständig nachgeregelt wird, so dass die von den mindestens zwei Sensoren ermittelten Messwertverläufe immer möglichst weitestgehend bis auf genau diese Verzögerung gleich sind.

Für die von den Sensoren ausgesendeten Lichtpulse kann (zumindest innerhalb eines Messvorgangs) eine gleichbleibende Dauer von beispielsweise ca. 1 ms vorgesehen sein. Vorteilhaft kann jedoch sein, wenn in Phasen eines Messwertanstiegs die Dauer der Lichtpulse im Vergleich zu Phasen eines Messwertabfalls verlängert gewählt wird. In Verbindung mit einer weiterhin bevorzugten mehrfachen oder länger (als in den anderen Phasen) andauernden Detektion des reflektierten und/oder transmittierten Anteils des Lichts je Lichtpuls während eines Messwertanstiegs kann dadurch ohne übermäßigen Energieaufwand und unzulässige Erwärmung der Sensoren eine möglichst hohe Auflösung während des Messwertanstiegs und damit während desjenigen Abschnitts der einzelnen Messwertverläufe, die vorzugsweise die zur Ermittlung des Zeitversatzes genutzten charakteristischen Werte umfassen, erreicht werden. Dadurch kann sich zwar eine zeitliche Verzerrung der Messwerte über der Zeit und unter Umständen auch der Messwertamplituden ergeben, was jedoch die erfindungsgemäße Auswertung der Messwertverläufe nicht behindert. Im Gegenteil können dadurch vorteilhaft besonders relevante Phasen (die betrachteten charakteristischen Werte) der Messwertverläufe stärker gewichtet werden.

Weiterhin, wiederum um die vom systolischen Druckanstieg verursachte schnelle Signaländerung zeitlich möglichst hoch aufzulösen, kann in einer bevorzugten Ausführungsform des erfindungsgemäßen Verfahrens vorgesehen sein, dass zur Ermittlung von Messwerten nicht abgewartet wird, bis Einschwingvorgänge in den Detektoren beendet sind und somit im Wesentlichen konstante Messsignalwerte abgelesen werden können. Vielmehr erfolgt eine Ermittlung der Messwerte anhand einer Auswertung (ggf. jeweils eines Abschnitts) der Einschwingvorgänge, insbesondere durch Ermittlung der maximalen Steigung und/oder des Integrals des Messsignalverlaufs.

In einer weiterhin bevorzugten Ausführungsform des erfindungsgemäßen Verfahrens kann vorgesehen sein, dass die mittels der Klemmdruckmessung erfolgte Messung des Blutdrucks nicht nur zur Kalibrierung der sensorbasierten Messwertermittlungen herangezogen wird, sondern umgekehrt auch die Messung des Blutdrucks mittels der Klemmdruckmessung durch die gleichzeitig erfolgende, sensorbasierte Messwertermittlung verbessert wird. Demnach kann auch vorgesehen sein, dass während der Kalibrierungsmessung sensorbasierte Messwertverläufe ermittelt und ein mittels der Kalibrierungsmessung bestimmter Druckverlauf anhand dieser Messwertverläufe angepasst wird.

Durch die Integration der Sensoreinheit in die Druckmanschette kann diese vorteilhaft dazu genutzt werden, um die Sensoren auch dann sicher und insbesondere mit einer definierten Andrückkraft an den vorgesehenen Stellen der zur Bestimmung des Blutdrucks genutzten Extremität zu halten, wenn keine Klemmdruckmessung durchgeführt wird. Dazu kann vorgesehen sein, dass dann, wenn keine Klemmdruckmessung durchgeführt wird, ein Halteklemmdruck in der Druckmanschette aufgebracht wird. Dieser Halteklemmdruck ist vorzugsweise so bemessen, dass die Haltefunktion erfüllt, gleichzeitig eine Beeinflussung des venösen Rückflusses in den Blutgefäßen jedoch möglichst gering gehalten oder vermieden wird. Beispielsweise kann der Halteklemmdruck ca. 5 bis 20 mmHg, insbesondere 10 mmHg betragen.

Um für die mindestens zwei Sensoren ein möglichst gleiches Einschwingverhalten für die Messsignale zu erhalten, kann vorzugsweise vorgesehen sein, dass beide Sensoren einen gemeinsamen Detektor oder zwei parallel geschaltete Detektoren aufweisen.

Um den Detektor eines Sensors möglichst vor Fremdlicht abzuschirmen, kann weiterhin vorgesehen sein, dass dieser zwischen dem Leuchtmittel des entsprechenden Sensors sowie einer in Umfangsrichtung der Extremität gerichteten Längsachse der Druckmanschette angeordnet ist. Bei zwei vorgesehenen Sensoren können sich somit die Detektoren zwischen den beiden außenliegenden Leuchtmitteln befinden.

Die Erfindung wird nachfolgend anhand eines in den Zeichnungen dargestellten Ausführungsbeispiels näher erläutert. In den Zeichnungen zeigt jeweils schematisch:
- Fig. 1:: die Verwendung einer erfindungsgemäßen Vorrichtung;
- Fig. 2:: ein Funktionsschaltbild zur Vorrichtung der Fig. 1;
- Fig. 3:: die Bestromung der zwei Leuchtdioden sowie der sich daraus ergebende Messsignalverlauf in einer Messsequenz;
- Fig. 4:: die zwei mittels der zwei Sensoren der Vorrichtung ermittelten Messwertverläufe;
- Fig. 5a:: eine Ausführungsform einer Druckmanschette der Vorrichtung gemäß der Fig. 1 im nicht befüllten Zustand; und
- Fig. 5b:: die Druckmanschette gemäß der Fig. 5a im befüllten Zustand.

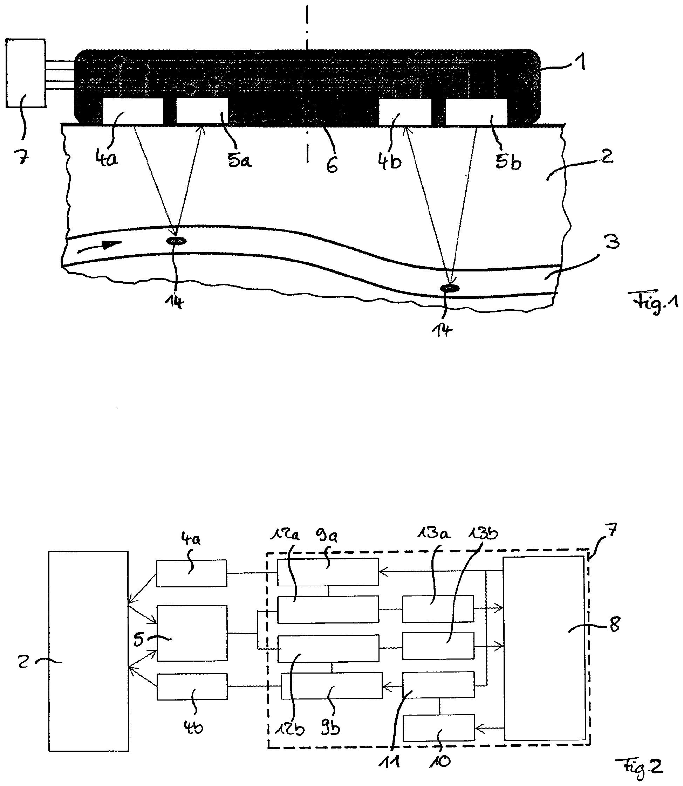

Die Fig. 1 zeigt schematisch eine erfindungsgemäße Vorrichtung zur Bestimmung des Blutdrucks in einem Blutgefäß, beispielsweise einer Arterie, eines Menschen oder Tieres. Die Vorrichtung umfasst eine Druckmanschette 1, die um eine Extremität 2, beispielsweise einen Oberarm eines Menschen, gelegt werden kann. Die Druckmanschette 1 kann variabel mit einem Fluid, beispielsweise Luft, befüllt werden, wodurch sich diese ausdehnt. Die damit einhergehende Verkleinerung des freien Innenquerschnitts der Druckmanschette 1 führt zu einem radialen Komprimieren des darin gelegenen Abschnitts der Extremität 2 und damit zur Ausübung eines Klemmdrucks auf einen in diesem Abschnitt der Extremität 2 verlaufenden Abschnitt einer Arterie 3. Mittels der Druckmanschette 1 wird in mehr oder weniger regelmäßigen Abständen in bekannter Weise eine Klemmdruckmessung durchgeführt. Diese dient der Ermittlung einer Transferfunktion sowie der Kalibrierung von sensorbasierten Messungen der Pulswellenlaufzeit und einer daraus mittels der Transferfunktion ermittelten Pulskurvenform des Blutdrucks. Die Klemmdruckmessung kann beispielsweise als oszillatorische Messung ausgeführt werden, bei der mittels eines den Innendruck der Druckmanschette 1 messenden Drucksensors (nicht dargestellt) Druckänderungen, die beim gezielten Absenken des Innendrucks durch eine einsetzende Blutströmung in der Arterie und die dadurch erzeugten Schwingungen erzeugt werden, gemessen werden. Anhand dieser Druckänderungen kann in bekannter Weise der quantitative Pulskurvenverlauf ermittelt werden.

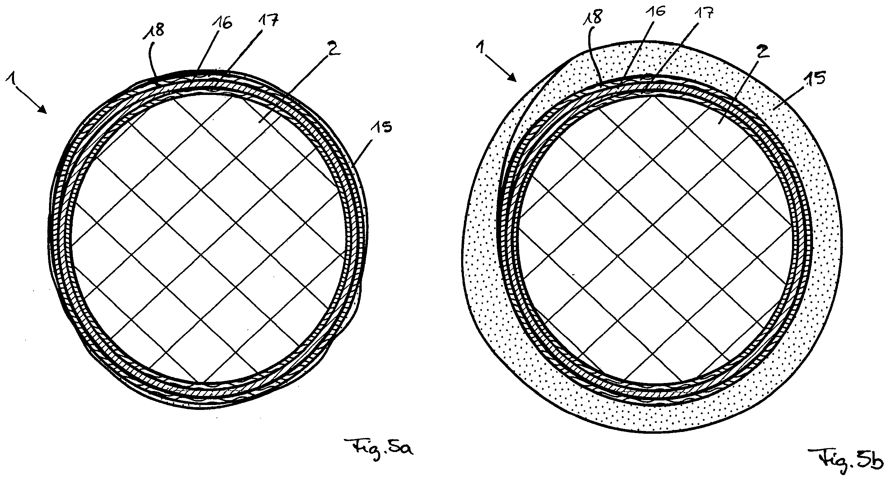

Die Druckmanschette 1 kann vorzugsweise, wie dies in den Fig. 5a und 5b dargestellt ist, einen mit dem Fluid befülllbaren ersten Manschettenteil 15 sowie ein zwischen dem ersten Manschettenteil 15 und der Extremität 2 angeordneten zweiten Manschettenteil 16 aufweisen, der derart ausgebildet ist, dass dieser die Ausbildung von Falten, insbesondere beim Befüllen des ersten Manschettenteils 15, vermeidet. Insbesondere kann der zweite Manschettenteil 16 aus einer Schicht eines relativ (bezogen auf den Werkstoff, aus dem der erste Manschettenteil 15 ausgebildet ist) steifen Materials, insbesondere Kunststoffmaterials, bestehen, und in seiner unbelasteten Ausgangsform spiralförmig aufgerollt mit sich überlappenden Enden ausgebildet sein. Der zweite Manschettenteil 16 ist dabei vorzugsweise derart elastisch deformierbar ausgebildet, dass dessen Spiralform zumindest ein Stück weit aufgebogen werden kann, was insbesondere für ein Anlegen der Druckmanschette 1 an die Extremität 2 vorteilhaft sein kann. Durch den zweiten Manschettenteil 16 kann eine möglichst gleichförmige Übertragung des von dem ersten Manschettenteil 15 bei der Befüllung mit dem Fluid erzeugten radialen Drucks auf den gesamten zwischen der Druckmanschette 1 und der Extremität 2 ausgebildeten Kontaktbereich bewirkt werden (vgl. Fig. 5b). Zudem kann die Rückstellkraft des elastisch aufgebogenen zweiten Manschettenteils 16 auch dann für ein sicheres und enges Anliegen der Druckmanschette 1 an der Extremität 2 sorgen, wenn der ersten Manschettenteil 15 nicht in einem Maße mit dem Fluid befüllt ist, dass von diesem ein relevanter radialer Druck auf die Extremität 2 ausgeübt wird (vgl. Fig. 5a).

Um den Tragekomfort der Druckmanschette 1 zu erhöhen, kann diese weiterhin noch einen dritten Manschettenteil 17 umfassen, der zwischen dem zweiten Manschettenteil 16 und der Extremität 2 angeordnet ist. Der dritte Manschettenteil 17 kann insbesondere aus einem weichen, flexiblen Material, beispielsweise einem Gewebe bestehen und/oder als in sich (ringförmig) geschlossene Manschette ausgebildet sein. Ein besonderer Vorteil eines solchen dritten Manschettenteils 17 kann darin liegen, dass dieser ein Einklemmen eines Teils der Oberfläche der Extremität 2 im Bereich der sich überlappenden Enden des zweiten Manschettenteils 17 verhindern kann. Sofern der dritte Manschettenteil 17 zudem radial elastisch aufweitbar ausgebildet ist, kann sichergestellt werden, dass dieser (gemeinsam mit den anderen Manschettenteilen) durch Aufweitung problemlos auf die Extremität 2 aufgezogen werden kann, danach beim Anliegen an der Extremität 2 aber ebenfalls (wie der zweite Manschettenteil 16) keine Falten ausbildet.

Es kann weiterhin ein vierter Manschettenteil 18 vorgesehen sein, der zwischen dem ersten Manschettenteil 15 und dem zweiten Manschettenteil 16 angeordnet ist und entsprechend dem dritten Manschettenteil 17 ausgebildet sein kann. Der vierte Manschettenteil 18 kann insbesondere eine Beschädigung des ersten Manschettenteils 15 durch die freien Enden des zweiten Manschettenteils 16 verhindern.

Eine solche Druckmanschette ist in der internationalen Patentanmeldung PCT/EP2014/000340 offenbart, deren gesamter Inhalt durch Bezugnahme zum Inhalt der vorliegenden Beschreibung gemacht wird.

In die Druckmanschette sind zwei optische Sensoren integriert, wie beispielsweise in der Fig. 1 dargestellt. Bei der bevorzugten Ausführung der Druckmanschette 1 mit mehreren Manschettenteilen (vgl. Fig. 5a und 5b) sind die Sensoren an der der Extremität 2 zugewandten Seite des zweiten Manschettenteils 16, oder, wenn ein dritter Manschettenteil 17 vorgesehen ist, an der entsprechenden Seite dieses dritten Manschettenteils 17 angeordnet.

Jeder der Sensoren umfasst ein Leuchtmittel in Form einer Leuchtdiode 4a, 4b (LED) sowie einen benachbart angeordneten Detektor in Form einer Photodiode 5a, 5b (PD). Durch die Anordnung unterhalb der vorzugsweise weitgehend lichtundurchlässigen Druckmanschette 1 werden die Photodioden 5a, 5b bereits gut vor einer Einstrahlung von Fremdlicht geschützt. Die Anordnung der Photodioden 5a, 5b jeweils zwischen einer in Umfangsrichtung gerichteten Mittellängsachse 6 der Druckmanschette 1 sowie der jeweils dazugehörigen Leuchtdiode 4a, 4b sorgt zudem dafür, dass randseitig unter die Druckmanschette 1 einstrahlendes Licht die Photodioden 5a, 5b möglichst nicht erreicht. Die Leuchtdioden 4a, 4b sind antiparallel geschaltet, um die Anzahl der Anschlussleitungen gering zu halten.

Die Photodioden 5a, 5b sind parallel verschaltet und elektrisch mit einer Auswerteeinheit 7 der Vorrichtung verbunden. Durch die parallele Verschaltung wirken beide Photodioden 5a, 5b elektrisch wie eine einzelne Photodiode. Messsignale, die die Photodioden 5a, 5b aufgrund eines Lichteinfalls erzeugen, durchlaufen somit prinzipiell die gleiche Empfangsschaltung. Dadurch wird erreicht, dass das Einschwingverhalten für die Messsignale beider Photodioden 5a, 5b im Wesentlichen gleich ist.

Alternativ zu der hier dargestellten Ausgestaltung mit zwei parallel verschalteten Photodioden 5a, 5b kann auch eine für beide Sensoren gemeinsam genutzte Photodiode 5 eingesetzt werden (in der Fig. 2 schematisch dargestellt). Eine solche gemeinsame Photodiode 5 erzeugt Messsignale aufgrund von Lichteinstrahlungen, die auf Lichtpulsen beruhen, die von beiden Leuchtdioden 4a, 4b ausgestrahlt wurden. Eine solche gemeinsame Nutzung ist wegen des definierten Zeitversatzes, mit der die zwei Leuchtdioden 4a, 4b nacheinander aktiviert werden, problemlos möglich. Eine gemeinsame Photodiode 5 könnte beispielsweise mittig zwischen den beiden Leuchtdioden 4a, 4b angeordnet sein.

Zur Ermittlung der Pulswellenlaufzeit werden die beiden Leuchtdioden 4a, 4b von der Auswerteeinheit 7 (vgl. Fig. 1) gepulst angesteuert, wodurch diese in dem von der Ansteuerung vorgegebenen Takt Lichtpulse in das unterhalb der Sensoren liegende Gewebe sowie das durch die Arterie 3 strömende Blut ausstrahlen. Dabei wird ein Teil des Lichts von dem Gewebe und dem Blut reflektiert, wobei ein Anteil davon in die jeweils dazugehörige oder gemeinsame Photodiode 5, 5a, 5b einstrahlt. In der Fig. 1 ist beispielhaft jeweils ein Lichtstrahl dargestellt, der von einem Blutkörperchen 14 in die Photodiode 5a, 5b reflektiert wird. Tatsächlich erfolgt eine solche Reflektion von einer Vielzahl von Blutkörperchen 14 und auch von dem die Arterienwand ausbildenden und umgebenden Gewebe. Da sich das Gewebe jedoch kurzfristig nicht relevant verändert, stellt sich der von diesem reflektierte Anteil des Lichts bei kurz aufeinander folgenden Messsignalermittlungen als Gleichanteil dar, während Veränderungen (Wechselanteile im Bereich der Herzfrequenz) primär auf den Anteil des von dem Blut reflektierten Lichts zurück zu führen sind. Diese Veränderungen sind primär darin begründet, dass die herzschlagbedingten Druckwellen, die sich mit der Pulswellengeschwindigkeit durch die Arterie 3 bewegen, zu einem zyklischen, druckbedingten, lokalen Aufweiten der Arterie 3 führen und sich strömungsbedingt die Orientierung und Form der Blutkörperchen ändert.

Wie sich aus der Fig. 2 ergibt, umfasst die Auswerteeinheit 7 eine Recheneinheit 8 und jeweils einen Timer 9a, 9b für jede der Leuchtdioden 4a, 4b. Die Leuchtdioden 4a, 4b werden nach Vorgabe der Recheneinheit 8 mittels der Timer 9a, 9b angesteuert. Zwischen den Timer 9b der zweiten, bezogen auf die Strömungsrichtung des Bluts in der Arterie stromab angeordneten Leuchtdiode 4b und die Recheneinheit 8 ist noch ein mittels eines Reglers 10 gesteuertes Verzögerungselement 11 geschaltet. Die zweite Leuchtdiode 4b wird somit erst durch das Verzögerungselement 11 mit einem von der Recheneinheit 8 vorgegebenen Zeitversatz nach einer Ansteuerung der ersten Leuchtdiode 4a angesteuert. Die Photodiode(n) 5a, 5b sind über zwei Integratoren 12a, 12b und Analog-Digital-Wandler 13a, 13b mit der Recheneinheit verbunden. Die in der Fig. 2 gezeigten Timer 9a, 9b, das Verzögerungselement 11, der Regler 10, die Integratoren 12a, 12b und die Analog-Digital-Wandler 13a, 13b sind funktionale Elemente, die auch in die Recheneinheit 8 integriert sein können beziehungsweise deren Funktion auch von der Recheneinheit 8 übernommen werden kann.

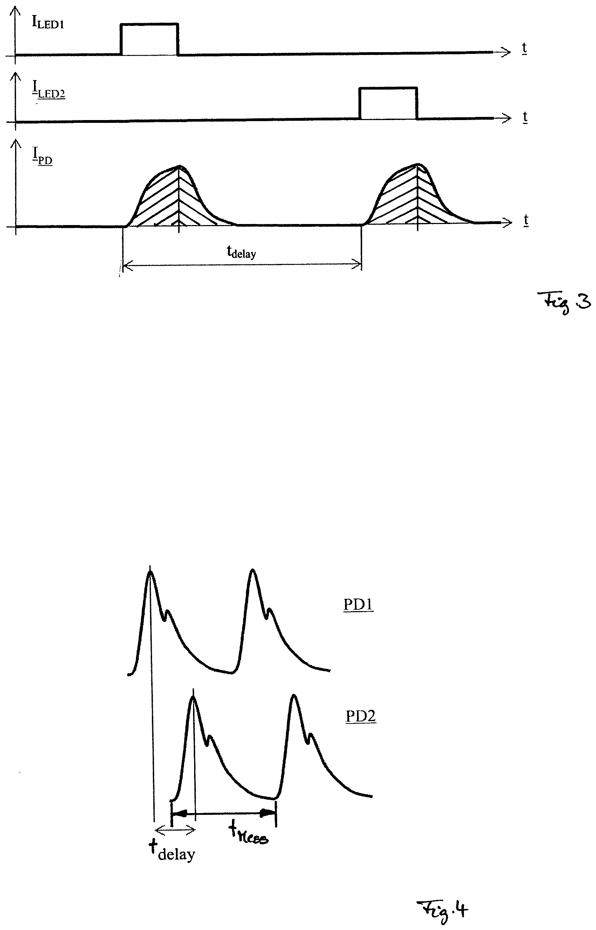

Ein Ablauf einer mittels der beiden Sensoren durchgeführten Messsequenz, deren Gesamtdauer beispielsweise 1 ms betragen kann, läuft folgendermaßen ab. Mittels der gemeinsamen oder der zu der ersten Leuchtdiode 4a zugehörigen Photodiode 5a wird eine Dunkelstrommessung durchgeführt. Dies dient dazu, den durch Fremdlichteinstrahlung begründeten Messfehler während einer darauffolgenden Messsignalerfassung bei angesteuerter erster Leuchtdiode 4a zu bestimmen und herausrechnen zu können. Daraufhin erfolgt eine pulsartige Ansteuerung der ersten Leuchtdiode 4a mit einem Ansteuerstrom (vgl. Fig. 3: I_{LED1}), was zu einem Ausstrahlen eines Lichtpulses durch die erste Leuchtdiode 4a führt. Ein Anteil des dabei ausgestrahlten Lichts wird von der (dazugehörigen) Photodiode 5, 5a detektiert und in ein entsprechendes Messsignal (I_{PD}) gewandelt. Wegen des Einschwingverhaltens der Photodiode 5, 5a und der damit verschalteten Empfangsschaltung stellt sich das Messsignal nicht digital als mit einem Endwert "vorhanden" oder "nicht vorhanden" ein, sondern es ist ein relativ langsamer Anstieg von Null (beziehungsweise des als Null definierten, bei der Dunkelstrommessung ermittelten Messsignals) erkennbar (vgl. Fig. 3). Die Pulsdauer der Ansteuerung der ersten Leuchtdiode 4a ist so kurz bemessen, dass diese wieder ausgeschaltet wird, noch bevor das Messsignal der (dazugehörigen) Photodiode 5, 5a überhaupt eingeschwungen ist und somit ein im Wesentlichen konstantes Signal liefert. Als Messwert für den entsprechenden Lichtpuls kann somit nicht ein eingeschwungenes Messsignal dienen. Vielmehr wird typischerweise das Integral unter der Messsignalkurve über dem Zeitraum des Lichtpulses (rechtsschraffierte Fläche in der Fig. 3) genutzt. Möglich und vorteilhaft kann aber auch sein, das Integral über die gesamte Zeitspanne zu bilden, in der das Messsignal von Null unterschiedlich ist (ca. doppelte Lichtpulsdauer; Summe aus rechts- und linksschraffierter Fläche in der Fig. 3). Ein so erhaltener Messwert für den betrachteten Lichtpuls der ersten Leuchtdiode 4a wird mittels des dazugehörigen Analog-Digital-Wandlers 13a gewandelt und der Recheneinheit 8 zugeführt.

Nach einem definierten Zeitversatz (t_{delay}), der durch das Verzögerungselement 11 eingestellt wird, wird die zweite Leuchtdiode 4b entsprecht pulsartig angesteuert. Eine Messwertermittlung erfolgt entsprechend derjenigen bei der ersten Leuchtdiode 4a.

Die zwei innerhalb einer Messsequenz ermittelten Messwerte, einer für jeden der Sensoren, sind abhängig von den jeweils vorliegenden Volumina, die von den unter den Sensoren liegenden Abschnitten der Arterie 3 ausgebildet werden. Diese Volumina und damit die Messwerte verändern sich infolge der mit der Pulswellengeschwindigkeit durch die Arterie 3 wandernden, herzschlagbedingten Druckwellen. Sofern eine Vielzahl von entsprechenden Messsequenzen hintereinander durchgeführt wird, kann aus den Messwerten jedes der beiden Sensoren jeweils ein Messwertverlauf und in Verbindung mit der Transferfunktion jeweils eine Pulskurvenform abgeleitet werden (vgl. Fig. 4). Diese Pulskurvenformen sind, gegebenenfalls nach einer Normierung, weitgehend identisch, jedoch um den Zeitversatz (t_{delay}) versetzt.

Sofern der Zeitversatz zwischen der Ansteuerung der beiden Leuchtdioden 4a, 4b derart ist, dass die in einer Messsequenz ermittelten Messwerte relativ gesehen gleich sind, d.h. im Wesentlichen dieselbe Stelle auf den zwei normierten Pulskurvenformen darstellen, entspricht der Zeitversatz der (Pulswellen-)Laufzeit, die die Pulswellen für die Wanderung von dem ersten zu dem zweiten Sensor benötigen. In Verbindung mit dem bekannten Abstand der zwei Sensoren (hier kann näherungsweise der Abstand zwischen den beiden Sensoren angesetzt werden, z.B. von ca. 5 bis 10 cm, insbesondere ca. 8 cm) kann aus der Pulswellenlaufzeit die Pulswellengeschwindigkeit ermittelt werden.

Bekannt ist, dass die Pulswellengeschwindigkeit vom arteriellen Blutdruck abhängig ist. Konkret ist die Pulswellengeschwindigkeit von der Steifigkeit der arteriellen Gefäßwände abhängig, die wiederum druckabhängig ist. Insbesondere kann bei kurzzeitiger Anwendung mit entsprechend geringen Änderungen (des Blutmitteldrucks) die Laufzeitänderung als umgekehrt proportional zu der Änderung des Blutmitteldrucks (MAP) angesehen werden. Bei größeren Veränderungen kann immerhin noch eine funktionale Abhängigkeit von dem Blutmitteldruck angenommen werden. Demnach kann mittels der ermittelten Pulswellengeschwindigkeit auf den Blutmitteldruck geschlossen werden.

Über die Transferfunktion, die unter Nutzung des Ergebnisses einer Klemmdruckmessung erhalten wurde, kann der sensorbasiert ermittelte Blutmitteldruck in eine quantitative Pulskurvenform übertragen werden. Mittels der Messsignale kann somit kontinuierlich der Blutdruckverlauf bestimmt werden, wobei vorgesehen ist, in definierten Zeitintervallen jeweils eine Klemmdruckmessung durchzuführen, um eine Neukalibrierung der Messsignale beziehungsweise des daraus abgeleiteten Blutdruckverlaufs zu ermöglichen.

Ein solches Zeitintervall, das beispielsweise ca. 5 Minuten betragen kann, umfasst demnach eine Vielzahl von Messvorgängen. Jeder Messvorgang (t_{Mess}) umfasst (zumindest) die Ermittlung eines Zyklus der Messwertverläufe beider Sensoren. Da diese durch die kontinuierliche Phasenregelung mit dem Herzschlag synchronisiert sind, beträgt die Dauer eines Messvorgangs ca. 0,25 s bis 2s (entsprechend einer Herzschlagfrequenz von 30 bis 240, zuzüglich des nur geringfügig ins Gewicht fallenden, eingestellten Zeitversatzes, der der Pulswellenlaufzeit entspricht und bei einer Sensordistanz von ca. 8 cm ca. 6,75 bis 10 ms beträgt).

Die Regelung des Zeitversatzes, mit dem in jeder Messsequenz die zweite Leuchtdiode 4b nach der ersten Leuchtdiode 4a angesteuert wird, erfolgt mindestens einmal für jeden Messvorgang. Dabei wird für eine oder mehrere Messsequenzen des gerade ablaufenden Messvorgangs entschieden, ob die entspreche(n) Messsequenz(en) des vorherigen Messvorgangs zu im Wesentlichen gleichen relativen Messwerten geführt hat/haben. Ist eine Abweichung erkennbar, wird der bei dem vorherigen Messvorgang genutzte Zeitversatz entsprechend angepasst. Dieser angepasste Zeitversatz wird dann den Messsequenzen des neuen beziehungsweise eines diesem nachfolgenden Messvorgangs zugrunde gelegt.

Ein Verfahren nach der vorliegenden Erfindung zur Blutdruckermittlung ist typischerweise kein diagnostisches Verfahren. Es erlaubt keine finale Diagnose eines Krankheitsbildes, sondern dient dazu, die Funktionstüchtigkeit des kardiovaskulären Systems eines Probanden oder Patienten zu bestimmen. Ermittelte Abweichungen vom Blutdruck einer als gesund anerkannten Person, die pathologischer oder nicht-pathologischer Natur, bspw. bei erregten Probanden, sein können, sind jedoch nur Anhaltspunkte für ggf. weitere erforderliche diagnostische Abklärungen.

### Bezugszeichenliste

- 1: Druckmanschette
- 2: Extremität
- 3: Arterie
- 4a: Leuchtdiode des ersten Sensors
- 4b: Leuchtdiode des zweiten Sensors
- 5a: Photodiode des ersten Sensors
- 5b: Photodiode des zweiten Sensors
- 6: Mittellängsachse
- 7: Auswerteeinheit
- 8: Recheneinheit
- 9a: Timer des ersten Sensors
- 9b: Timer des zweiten Sensors
- 10: Regler
- 11: Verzögerungselement
- 12a: Integrator des ersten Sensors
- 12b: Integrator des zweiten Sensors
- 13a: Analog-Digital-Wandler des ersten Sensors
- 13b: Analog-Digital-Wandler des zweiten Sensors
- 14: Blutkörperchen
- 15: erster Manschettenteil
- 16: zweiter Manschettenteil
- 17: dritter Manschettenteil
- 18: vierter Manschettenteil

## Patentansprüche

1. Verfahren zur Bestimmung des Blutdrucks in einem Blutgefäß, wobei in einem Messvorgang mittels mindestens zweier in einem definierten Abstand zueinander angeordneten Sensoren (4a, 4b, 5, 5a, 5b)
eine Pulswellenlaufzeit ermittelt wird, wobei durch eine Kalibrierung, insbesondere mittels einer Klemmdruckmessung, aus einer Pulswellenlaufzeitänderung der Blutdruck ohne Verwendung eines weiteren, zur Strömungsgeschwindigkeit proportionalen Signals ermittelt wird, wobei die Pulswellenlaufzeitänderung umgekehrt proportional zur Blutdruckänderung ist,
wobei mittels eines Leuchtmittels (4a, 4b) der Sensoren Lichtpulse in Richtung des Blutgefäßes ausgestrahlt werden, wobei mittels eines Detektors (5, 5a, 5b) der Sensoren ein von dem Blut in dem Blutgefäß reflektierter Anteil und/oder ein durch das Blut in dem Blutgefäß transmittierter Anteil des Lichtpulses detektiert wird, wobei ein Zeitversatz zwischen der Aktivierung der zwei Sensoren derart geregelt wird, dass der Zeitversatz der Pulswellenlaufzeit entspricht, so dass gleiche relative Messwerte erzielt werden, die im Wesentlichen die gleiche Stelle der hinsichtlich der Kurvenform vergleichbaren Messwertverläufe darstellen.

2. Verfahren gemäß Anspruch 1, **dadurch gekennzeichnet, dass** eine Mehrzahl von Messvorgängen durchgeführt wird, wobei Kalibrierungen jeweils nach mehreren Messvorgängen durchgeführt werden.

3. Verfahren gemäß einem der vorhergehenden Ansprüche, **dadurch gekennzeichnet, dass** der Zeitversatz anhand charakteristischer Werte der Messwertverläufe ermittelt wird.

4. Verfahren gemäß Anspruch 3, **dadurch gekennzeichnet, dass** als charakteristische Werte die Minima, Maxima und/oder Werte an Stellen des maximalen Messwertanstiegs herangezogen werden.

5. Verfahren gemäß einem der vorhergehenden Ansprüche, **dadurch gekennzeichnet, dass** in Phasen eines Messwertanstiegs die Dauer der Lichtpulse im Vergleich zu Phasen eines Messwertabfalls verlängert gewählt wird.

6. Verfahren gemäß Anspruch 5, **dadurch gekennzeichnet, dass** in Phasen eines Messwertanstiegs mehrere Detektionen je Lichtpuls durchgeführt werden.

7. Verfahren gemäß Anspruch 5 oder 6, **dadurch gekennzeichnet, dass** in Phasen eines Messwertanstiegs die Dauer der Detektion im Vergleich zu Phasen eines Messwertabfalls verlängert gewählt wird.

8. Verfahren gemäß einem der vorhergehenden Ansprüche, **dadurch gekennzeichnet, dass** die Anzahl der Lichtpulse je Herzschlag in Abhängigkeit von der Herzfrequenz konstant gehalten wird.

9. Verfahren gemäß einem der vorhergehenden Ansprüche, **dadurch gekennzeichnet, dass** zur Ermittlung eines Messwerts die maximale Steigung und/oder das Integral des entsprechenden Messsignalverlaufs genutzt wird.

10. Verfahren gemäß Anspruch 2 oder einem der von Anspruch 2 abhängigen Ansprüche, **dadurch gekennzeichnet, dass** dann, wenn keine Klemmdruckmessung durchgeführt wird, ein Halteklemmdruck mittels einer die Sensoren aufnehmenden Druckmanschette aufgebracht wird.

11. Vorrichtung zur Durchführung eines Verfahrens gemäß einem der vorhergehenden Ansprüche mit mindestens zwei Sensoren (4a, 4b, 5, 5a, 5b), einer Druckmanschette (1) zur Durchführung der Klemmdruckmessung, und einer Auswerteeinheit (7), wobei die Sensoren (4a, 4b, 5, 5a, 5b) in die Druckmanschette (1) integriert sind, wobei die Auswerteeinheit (7) angepasst ist, den Blutdruck in einem Blutgefäß zu bestimmen, wobei in einem Messvorgang mittels der mindestens zwei in einem definierten Abstand zueinander angeordneten Sensoren eine Pulswellenlaufzeit ermittelt wird, wobei durch eine Kalibrierung, insbesondere mittels einer Klemmdruckmessung, aus einer Pulswellenlaufzeitänderung der Blutdruck ohne Verwendung eines weiteren, zur Strömungsgeschwindigkeit proportionalen Signals ermittelt wird, wobei die Pulswellenlaufzeitänderung umgekehrt proportional zur Blutdruckänderung ist und die Auswerteeinheit (7) angepasst ist, die Vorrichtung so anzusteuern, dass mittels eines Leuchtmittels (4a, 4b) der Sensoren Lichtpulse in Richtung des Blutgefäßes ausgestrahlt werden, dass mittels eines Detektors (5, 5a, 5b) der Sensoren ein von dem Blut in dem Blutgefäß reflektierter Anteil und/oder ein durch das Blut in dem Blutgefäß transmittierter Anteil des Lichtpulses detektiert wird und dass ein Zeitversatz zwischen der Aktivierung der zwei Sensoren derart geregelt wird, dass der Zeitversatz der Pulswellenlaufzeit entspricht, so dass gleiche relative Messwerte erzielt werden, die im Wesentlichen die gleiche Stelle der hinsichtlich der Kurvenform vergleichbaren Messwertverläufe darstellen.

12. Vorrichtung gemäß Anspruch 11 zur Durchführung eines Verfahrens gemäß einem der Ansprüche 1 bis 10, **dadurch gekennzeichnet, dass** die Sensoren einen gemeinsamen Detektor oder zwei parallel geschaltete Detektoren aufweisen.

13. Vorrichtung gemäß Anspruch 12 zur Durchführung eines Verfahrens gemäß einem der Ansprüche 1 bis 10, **dadurch gekennzeichnet, dass** der Detektor eines Sensors zwischen dem Leuchtmittel dieses Sensors sowie einer Mittellängsachse (6) der Druckmanschette (1) angeordnet ist.

## Claims

1. A method for determining blood pressure in a blood vessel, wherein a pulse wave transit time is determined in a measurement process by means of at least two sensors (4a, 4b, 5, 5a, 5b) arranged at a defined distance from one another,
wherein the blood pressure is determined from a change in pulse wave transit time by calibration, in particular by means of a clamping pressure measurement, without using a further signal proportional to the flow velocity, wherein the change in pulse wave transit time is inversely proportional to the change in blood pressure, wherein light pulses are emitted in the direction of the blood vessel by means of an lighting means (4a, 4b) of the sensors, wherein a portion of the light pulse reflected by the blood in the blood vessel and/or a portion of the light pulse transmitted through the blood in the blood vessel is detected by means of a detector (5, 5a, 5b) of the sensors, wherein a time offset between the activation of the two sensors is regulated in such a way that the time offset corresponds to the pulse wave transit time, so that the same relative measured values are achieved which substantially represent the same point in the measured value profiles that are comparable in terms of the curve shape.

2. The method according to claim 1, **characterised in that** a plurality of measurement processes are carried out, wherein calibrations are carried out after a plurality of measurement processes.

3. The method according to any one of the preceding claims, **characterised in that** the time offset is determined based on characteristic values of the measured value profiles.

4. The method according to claim 3, **characterised in that** the minima, maxima and/or values at points of the maximum measured value are used as characteristic values.

5. The method according to any one of the preceding claims, **characterised in that** the duration of the light pulses is selected to be longer in phases when the measured value increases in comparison to phases when the measured value decreases.

6. The method according to claim 5, **characterised in that** a plurality of detections per light pulse are carried out in phases when the measure value increases.

7. The method according to claim 5 or 6, **characterised in that** the duration of the detection is selected to be longer in phases when the measured value increases in comparison to phases when the measured value decreases.

8. The method according to any one of the preceding claims, **characterised in that** the number of light pulses per heartbeat is kept constant as a function of the heart rate.

9. The method according to any one of the preceding claims, **characterised in that** the maximum gradient and/or the integral of the corresponding measurement signal profile is used to determine a measurement value.

10. The method according to claim 2 or one of the claims dependent on claim 2, **characterised in that** when no clamping pressure measurement is carried out, a holding clamping pressure is applied by means of a pressure cuff receiving the sensors.

11. A device for carrying out a method according to any one of the preceding claims, having at least two sensors (4a, 4b, 5, 5a, 5b),
a pressure cuff (1) for carrying out the clamping pressure measurement, and an evaluation unit (7), wherein the sensors (4a, 4b, 5, 5a, 5b) are integrated into the pressure cuff (1), wherein the evaluation unit (7) is adapted to determine the blood pressure in a blood vessel, wherein a pulse wave transit time is determined in a measurement process by means of the at least two sensors arranged at a defined distance from one another, wherein the blood pressure is determined from a change in pulse wave transit time by a calibration, in particular by means of a clamping pressure measurement, without using a further signal proportional to the flow velocity, wherein the change in pulse wave transit time is inversely proportional to the change in blood pressure, and the evaluation unit (7) is adapted to control the device in such a way that light pulses are emitted in the direction of the blood vessel by means of an lighting means (4a, 4b) of the sensors, so that a portion of the light pulse reflected by the blood in the blood vessel and/or a portion of the light pulse transmitted through the blood in the blood vessel is detected by means of a detector (5, 5a, 5b) of the sensors, and so that a time offset between the activation of the two sensors is regulated in such a way that the time offset corresponds to the pulse wave transit time, so that the same relative measured values are achieved which substantially represent the same point in the measured value profiles that are comparable in terms of the curve shape.

12. The device according to claim 11 for carrying out a method according to any one of claims 1 to 10, **characterised in that** the sensors have a common detector or two detectors connected in parallel.

13. The device according to claim 12 for carrying out a method according to any one of claims 1 to 10, **characterised in that** the detector of a sensor is arranged between lighting means of this sensor and a central longitudinal axis (6) of the pressure cuff (1) .

## Revendications

1. Procédé de détermination de la pression sanguine dans un vaisseau sanguin, dans lequel, lors d'un processus de mesure au moyen d'au moins deux capteurs (4a, 4b, 5, 5a, 5b) disposés à une distance définie l'un de l'autre,
un temps de propagation d'onde pulsatile est déterminé, dans lequel, par étalonnage, en particulier au moyen d'une mesure de pression de serrage, la pression sanguine est déterminée, sans utiliser un autre signal proportionnel à la vitesse d'écoulement, à partir d'une variation de temps de propagation d'onde pulsatile, dans lequel la variation du temps de propagation d'onde pulsatile est inversement proportionnelle à la variation de la pression artérielle, dans lequel, au moyen d'une source lumineuse (4a, 4b) des capteurs, des pulses lumineux sont émis dans la direction du vaisseau sanguin, dans lequel, au moyen d'un détecteur (5, 5a, 5b) des capteurs, une partie du pulse lumineux réfléchie par le sang dans le vaisseau sanguin et/ou une partie du pulse lumineux transmise à travers le sang dans le vaisseau sanguin est détectée, dans lequel un décalage temporel entre l'activation des deux capteurs est régulé de telle sorte que le décalage temporel corresponde au temps de propagation d'onde pulsatile de telle sorte que les mêmes valeurs mesurées relatives sont obtenues, lesquelles représentent essentiellement le même point des courbes de valeurs mesurées comparables en termes de forme de courbe.

2. Procédé selon la revendication 1, **caractérisé en ce qu'**une pluralité de processus de mesure sont mis en œuvre, dans lequel les étalonnages sont respectivement réalisés après plusieurs processus de mesure.

3. Procédé selon l'une quelconque des revendications précédentes, **caractérisé en ce que** le décalage temporel est déterminé en fonction des valeurs caractéristiques des courbes de valeurs mesurées.

4. Procédé selon la revendication 3, **caractérisé en ce que** les valeurs minimales, les valeurs maximales et/ou les valeurs aux points de l'augmentation maximale de la valeur mesurée sont utilisées comme valeurs caractéristiques.

5. Procédé selon l'une quelconque des revendications précédentes, **caractérisé en ce que**, dans les phases d'une augmentation de la valeur mesurée, la durée des pulses lumineux est choisie plus longue par rapport aux phases d'une diminution de la valeur mesurée.

6. Procédé selon la revendication 5, **caractérisé en ce que**, dans les phases d'une augmentation de la valeur mesurée, plusieurs détections par pulse lumineux sont réalisées.

7. Procédé selon la revendication 5 ou 6, **caractérisé en ce que**, dans les phases d'une augmentation de la valeur mesurée, la durée de la détection est choisie plus longue par rapport aux phases d'une diminution de la valeur mesurée.

8. Procédé selon l'une quelconque des revendications précédentes, **caractérisé en ce que** le nombre de pulses lumineux par battement cardiaque est maintenu constant en fonction de la fréquence cardiaque.

9. Procédé selon l'une quelconque des revendications précédentes, **caractérisé en ce que**, pour déterminer une valeur mesurée, l'augmentation maximale et/ou l'intégrale de la courbe de signal de mesure correspondante est utilisée.

10. Procédé selon la revendication 2 ou l'une quelconque des revendications dépendantes de la revendication 2, **caractérisé en ce que** lorsque aucune mesure de pression de serrage n'est réalisée, une pression de serrage de maintien est appliquée au moyen d'une manchette de pression logeant les capteurs.

11. Dispositif de mise en œuvre d'un procédé selon l'une quelconque des revendications précédentes comportant au moins deux capteurs (4a, 4b, 5, 5a, 5b), une manchette de pression (1) destinée à la réalisation de la mesure de pression de serrage et une unité d'évaluation (7), dans lequel les capteurs (4a, 4b, 5, 5a, 5b) sont intégrés dans la manchette de pression (1), dans lequel l'unité d'évaluation (7) est adaptée pour déterminer la pression sanguine dans un vaisseau sanguin, dans lequel, dans un processus de mesure au moyen des au moins deux capteurs disposés à une distance définie l'un de l'autre, un temps de propagation d'onde pulsatile est déterminé, dans lequel, par étalonnage, en particulier au moyen d'une mesure de pression de serrage, la pression sanguine est déterminée, sans utiliser un autre signal proportionnel à la vitesse d'écoulement, à partir d'une variation de temps de propagation d'onde pulsatile, dans lequel la variation du temps de propagation d'onde pulsatile est inversement proportionnelle à la variation de la pression sanguine et l'unité d'évaluation (7) est adaptée pour commander le dispositif de telle sorte que, au moyen d'une source lumineuse (4a, 4b) des capteurs, des pulses lumineux sont émis dans la direction du vaisseau sanguin, que, au moyen d'un détecteur (5, 5a, 5b) des capteurs, une partie du pulse lumineux réfléchie par le sang dans le vaisseau sanguin et/ou une partie du pulse lumineux transmise à travers le sang dans le vaisseau sanguin est détectée, et qu'un décalage temporel entre l'activation des deux capteurs est régulé de telle sorte que le décalage temporel corresponde au temps de propagation d'onde pulsatile de telle sorte que les mêmes valeurs mesurées relatives sont obtenues, lesquelles représentent essentiellement le même point des courbes de valeurs mesurées comparables en termes de forme de courbe.

12. Dispositif selon la revendication 11 de mise en œuvre d'un procédé selon l'une quelconque des revendications 1 à 10, **caractérisé en ce que** les capteurs comportent un détecteur commun ou deux détecteurs montés en parallèle.

13. Dispositif selon la revendication 12 de mise en œuvre d'un procédé selon l'une quelconque des revendications 1 à 10, **caractérisé en ce que** le détecteur d'un capteur est disposé entre le moyen lumineux dudit capteur, ainsi qu'un axe longitudinal médian (6) de la manchette de pression (1).
